# EUROPÄISCHE PATENTANMELDUNG

(11) **EP 2 306 095 A2**
(43) Veröffentlichungstag der Anmeldung: **06.04.2011**
(21) Anmeldenummer: 10006750.3
(22) Anmeldetag: 30.06.2010
(51) Int. Cl.: F24C 15/20

(54) **Luftreinigungseinrichtung**

(30) Priorität: 05.10.2009 DE 202009013432 U
(71) Anmelder: Homeier Küchentechnik GmbH, 93057 Regensburg (DE)
(72) Erfinder: Homeier, Maximilian, 93057 Regensburg (DE)
(74) Vertreter: Graf, Helmut

(57) **Zusammenfassung**

Die Erfindung betrifft eine Luftreinigungseinrichtung, insbesondere zur Belüftung einer Kochfläche, mit einem Haubenkörper (2) und einem am Haubenkörper (2) angeordneten Kamin (3) mit einer Wandung (3.1). Besonders vorteilhaft ist im Kamin (3) ein Aufnahmeraum (4) zur Aufnahme zumindest einer Pflanze vorgesehen und in der Wandung (3, 1) des Kamins zumindest eine Ausnehmung (5) eingebracht, durch die der Aufnahmeraum (4) von außen zugängig ist.

## Beschreibung

Die Erfindung betrifft eine Luftreinigungseinrichtung, insbesondere zur Belüftung einer Kochfläche gemäß dem Oberbegriff des Schutzanspruchs 1.

Derartige Luftreinigungseinrichtungen bestehend aus einem Haubenkörper und einem am Haubenkörper angeordneten Kamin sind hinreichend bekannt.

Des Weiteren zeichnet sich zunehmend bei Privathaushalten wie auch in der Gastronomie ein Trend zu hochwertigen Zutaten, insbesondere ein Trend zur Verwendung von frischen Kräutern ab. Diese Kräuter nehmen zum einen eine gewisse Aufstellfläche im Bereich der Küche ein, was insbesondere bei kleinen Küchen die Ablageflächen zusätzlich einschränkt, zum anderen benötigen diese zu ihrem Wachstum bestimmte Umgebungsbedingungen, insbesondere Befeuchtungs- und Lichtverhältnisse.

Ausgehend hiervon liegt der Erfindung die Aufgabe zugrunde, eine neuartige Luftreinigungseinrichtung anzugeben, die ein Züchten von Pflanzen unterstützt. Die Aufgabe wird ausgehend vom Oberbegriff des Patentanspruchs 1 durch dessen kennzeichnende Merkmale gelöst.

Der wesentliche Aspekt der erfindungsgemäßen Luftreinigungseinrichtung ist darin zu sehen, dass im Kamin ein Aufnahmeraum zur Aufnahme zumindest einer Pflanze ausgebildet ist und dass in der Wandung des Kamins zumindest eine Ausnehmung vorgesehen ist, durch die der Aufnahmeraum von außen zugänglich ist. Dadurch ist es möglich Pflanzen, vorzugsweise Kräuterpflanzen in diesen Aufnahmeraum einzustellen, so dass in der Küche stets frische Kräuter verfügbar sind. Hierdurch ergibt sich eine besonders platzsparende Aufstellung von Pflanzen im Küchenbereich.

In einem besonders bevorzugten Ausführungsbeispiel ist oberhalb des Aufnahmeraums eine Beleuchtungseinrichtung, insbesondere ein Pflanzlicht zur Förderung des Pflanzenwachstums angeordnet. Durch die Bestrahlung der Pflanzen wird auch bei für das Pflanzenwachstum ungünstigen Lichtbedingungen innerhalb der Küche ein gutes Gedeihen derselben erreicht.

Weiterhin vorteilhaft ist der Kamin rohrförmig ausgebildet und weist umfangsseitig mehrere Ausnehmungen auf, wobei die Ausnehmungen jeweils durch Stege voneinander getrennt sind.

Auch ist am Boden des Aufnahmeraumes ein schalenartiges Element zur Aufnahme von Wasser angeordnet, welches vorzugsweise eine Füllstandsanzeige aufweist.

Oberhalb des Aufnahmeraums kann eine Beleuchtungseinrichtung, insbesondere zur Förderung des Pflanzenwachstums, angeordnet sein, die aus einer Vielzahl von Leuchtdioden besteht. Hierbei kann besonders vorteilhaft zur Steuerung der Beleuchtungseinrichtung eine Zeitschaltuhr vorgesehen sein.

Die erfindungsgemäße Luftreinigungsvorrichtung ist weiterhin vorteilhaft für den Umluftbetrieb ausgebildet und umfasst eine Entkeimungseinrichtung, beispielsweise eine Entkeimungslampe.

Zudem ergeben sich Weiterbildungen, Vorteile und Anwendungsmöglichkeiten der Erfindung auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Nachfolgend wird die Erfindung an einem Ausführungsbeispiel anhand von mehreren Figuren näher beschrieben. Es zeigen:
- Fig. 1: beispielhaft eine neuerungsgemäße Luftreinigungseinrichtung in einer Seitendarstellung;
- Fig. 2: beispielhaft eine neuerungsgemäße Luftreinigungseinrichtung in einer perspektivischen, unterseitigen Darstellung;
- Fig. 3: beispielhaft eine neuerungsgemäße Luftreinigungseinrichtung in einer perspektivischen, oberseitigen Darstellung;

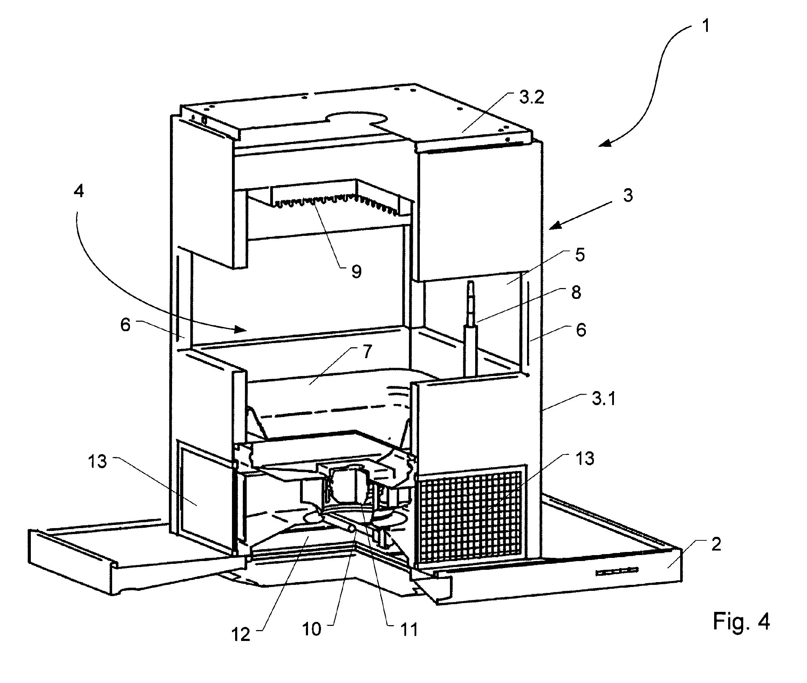

- Fig. 4: beispielhaft eine neuerungsgemäße Luftreinigungseinrichtung in einer perspektivischen Schnittdarstellung.

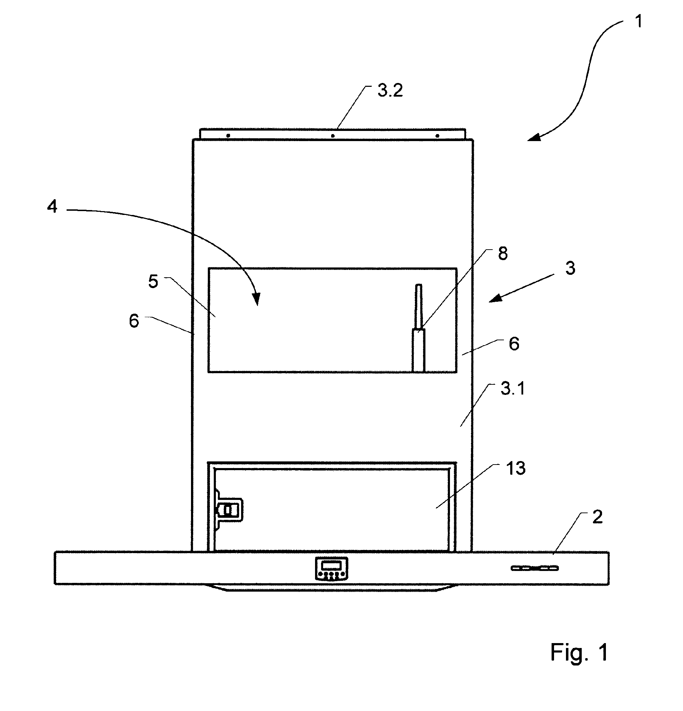

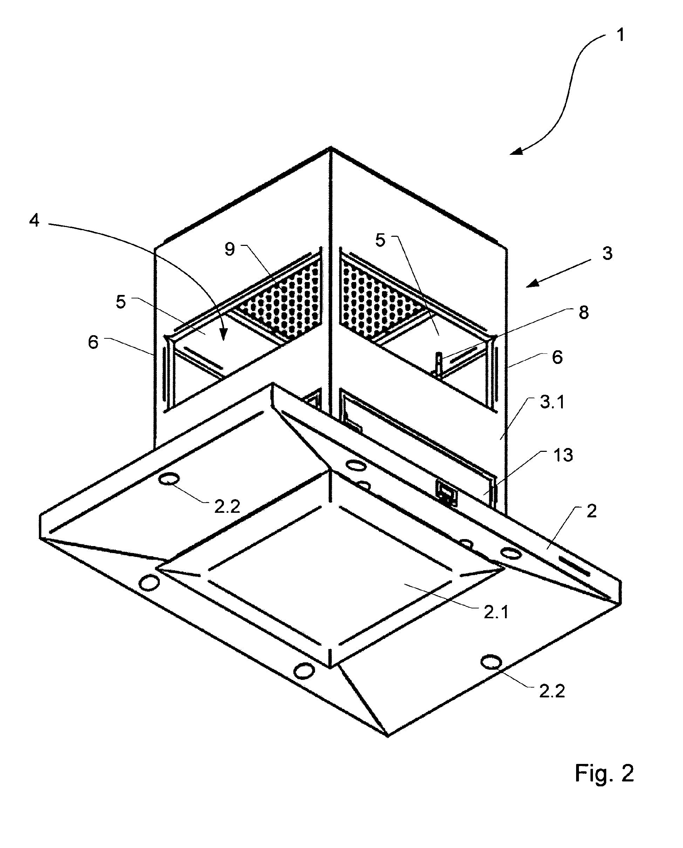

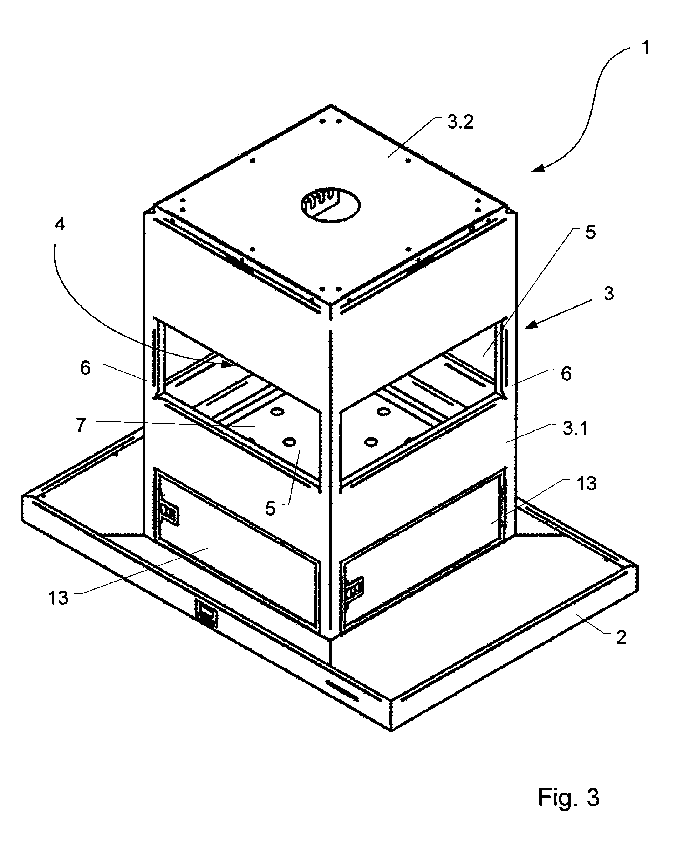

In den Figuren 1 - 3 sind jeweils erfindungsgemäße Luftreinigungseinrichtungen 1 in unterschiedlichen Darstellungen gezeigt. Die Luftreinigungseinrichtung 1 besteht in an sich bekannter Weise aus einem Haubenkörper 2 und einem am Haubenkörper 2 angeordneten, nach oben abstehenden Kamin 3.

Der Haubenkörper 2, der im gezeigten Ausführungsbeispiel im Querschnitt rechteckförmig ausgebildet ist, weist auf der zur belüftenden Kochfläche zugewandten Unterseite zumindest einen Fettfilter 2.1 und mehrere Leuchteinheiten 2.2 zur Beleuchtung der Kochfläche auf. Die vorzugsweise für den Umluftbetrieb ausgebildete Luftreinigungseinrichtung 1 weist im Inneren des Kamins 3 eine Lüftereinheit 11 auf, mittels derer die beim Kochen entstehenden Wrasen von der Kochfläche abgesaugt, über mehrere Filtereinheiten, insbesondere den Fettfilter 2.1, einen Aktivkohlefilter 12 und einen Pollenfilter 13 gereinigt und anschließend über in der Wandung 3.1 des Kamins 3 vorgesehenen Öffnungen an die Umgebungsluft wieder abgegeben werden.

Gemäß dem die Erfindung tragenden Gedanken ist im Inneren des Kamins 3 ein Aufnahmeraum 4 zur Aufnahme zumindest einer Pflanze ausgebildet, wobei dieser Aufnahmeraum 4 über Ausnehmungen 5 in der Wandung 3.1 des Kamins 3 von außen zugänglich ist.

Besonders bevorzugt ist der Kamin3 rohrförmig ausgebildet und weist umfangsseitig mehrere Ausnehmungen 5 in der Wandung 3.1 des Kamins auf, wobei die einzelnen Ausnehmungen 5 durch Stege 6 voneinander getrennt sind. Der Kamin 3 hat beispielsweise eine rechteckförmige oder quadratische Grundfläche, so dass die Wandung 3.1 des Kamins 3 vier Seitenflächen aufweist, wobei beispielsweise in jede der vier Seitenflächen eine Ausnehmung 5 eingebracht ist. Die Stege 6 sind vorzugsweise in den Eckbereichen des Kamins 3 vorgesehen.

Der Aufnahmeraum 4 ist im gezeigten Ausführungsbeispiel im Mittelbereich des Kamins 3 zwischen der Halterung 3.2, mittels dem die Luftreinigungseinrichtung 1 beispielsweise an einer Decke montierbar ist, und den Filtereinheiten, insbesondere den Pollenfiltern 13, über die die abgesaugte Luft an die Umgebungsluft wieder abgegeben wird, angeordnet. An der der Lüftereinheit 11 zugewandten Unterseite des Aufnahmeraums 4 ist ein schalenartiges Element, insbesondere ein Wasserbecken 7, vorgesehen, in das eine Füllstandsanzeige 8 eingebracht ist, um den Flüssigkeitsstand innerhalb des Wasserbeckens 7 anzuzeigen. Dadurch kann eine in dem Aufnahmeraum 4 aufgenommene Pflanze beispielsweise Kräuter, Blumen oder Ähnliches zumindest mit im Wurzelbereich in das Wasserbecken 7 eingestellt werden und durch die im Wasserbecken 7 aufgenommene Flüssigkeit, insbesondere Wasser befeuchtet werden. Mittels der Füllstandanzeige kann eine optimale Bewässerungssituation der im Aufnahmeraum 4 angeordneten Pflanzen sichergestellt werden.

Auf der dem Wasserbecken 7 gegenüberliegenden Seite des Aufnahmeraums 4 ist zumindest eine Beleuchtungseinrichtung 9 vorgesehen, die insbesondere zur Abgabe von Licht zur Förderung des Pflanzenwachstums ausgebildet ist. Die Beleuchtungseinrichtung 9 besteht u.a. vorzugsweise aus einer Vielzahl von Leuchtdioden, die sich durch eine geringe Leistungsaufnahme auszeichnen und die zur Abgabe von farbigem Licht, insbesondere rotem oder blauem Licht oder einer Kombination dieser Farben ausgebildet ist. Diese Farben werden von Pflanzen für die Photosynthese benötigt und fördern damit deren Wachstum. Aufgrund der äußerst geringen Wärmeabstrahlung der Leuchtdioden kann die Beleuchtungseinrichtung 9 sehr nahe an den Pflanzen angeordnet werden, ohne durch die entstehende Wärme das Pflanzenwachstum negativ zu beeinflussen.

In einem besonders bevorzugten Ausführungsbeispiel ist die Beleuchtungseinrichtung 9 mit einer Zeitschaltuhr ansteuerbar und dadurch zeitlich beliebig ein- bzw. ausschaltbar. Durch diese Zeitschaltuhr ist die Beleuchtungseinrichtung 9 nach beliebig programmierbaren Zeiten ein- bzw. ausschaltbar. Alternativ hierzu kann nach einem zeitgesteuerten Einschalten die Beleuchtungseinrichtung 9 nach einer gewissen Zeitdauer, in der die Beleuchtungseinrichtung 9 in Betrieb ist, ausgeschaltet werden oder durch eine Zufallsfunktion angesteuert werden, d.h. die Beleuchtungseinrichtung 9 wird für eine zufällige Dauer, insbesondere eine Dauer zwischen 5 und 20 Minuten einschaltet und bleibt nach dem Betrieb, beispielsweise zwischen 15 bis 60 Minuten wieder ausgeschaltet. Abweichend hiervon ist es selbstverständlich auch möglich, die Beleuchtungseinrichtung 9 manuell ein- bzw. auszuschalten.

Die in den Figuren gezeigte Luftreinigungseinrichtung 1 ist vorzugsweise für den Umluftbetrieb ausgebildet. Zur Reinigung der von der Kochfläche abgesaugten Luft, die im Allgemeinen mit Wasser und Fett angereichert ist, enthält die Luftreinigungseinrichtung 1 eine Entkeimungseinrichtung, vorzugsweise in Form einer Entkeimungslampe 10. Durch diese Entkeimungseinrichtung, die vorzugsweise eine UV-Entkeimungslampe ist, werden Krankheitserreger, wie Bakterien, Viren, Parasiten, Sporen, Schimmel und Staubmilben inaktiviert.

Durch die unmittelbar über dem Haubenkörper 2 angeordnete Lüftereinheit 11 und die zwischen dem Aufnahmeraum 4 und dem Haubenkörper 2 in die umfangsseitige Wandung des Kamins 3 angeordneten Filtereinheiten, insbesondere Pollenfilter 13, ergibt sich ein äußerst kompakter, eine geringe Bauhöhe aufweisender Aufbau der für den Betrieb der Lüftungseinrichtung 1 wesentlichen Komponenten, so dass ein großvolumiger und hoher Aufnahmeraum 4 zur Aufnahme der Pflanzen erreicht wird.

Durch die zusätzliche Auslegung der Lüftungseinrichtung 1 für den Umluftbetrieb kann sich der Aufnahmeraum 4 über die gesamte Grundfläche des Kamins 3 erstrecken. Abweichend hiervon ist es selbstverständlich auch möglich, die Luftreinigungseinrichtung 1 für den Abluftbetrieb auszubilden, wobei die Durchführung der abgesaugten Luft in Richtung der Längsachse des Kamins 3 durch einen bestimmten Teilbereich der Grundfläche des Kamins 3 erfolgen kann. Der durch die Luftabführung nicht belegte Teil der Grundfläche kann, wie bei einer Luftreinigungseinrichtung 1 im Umluftbetrieb, für einen Aufnahmeraum 4 für Pflanzen verwendet werden.

Die Erfindung wurde voranstehend an einem Ausführungsbeispiel beschrieben. Es versteht sich, dass zahlreiche Modifikationen und Änderungen der Erfindung möglich sind, ohne dass hierdurch der Erfindungsgedanke verlassen wird.

### Bezugszeichenliste

- 1: Luftreinigungseinrichtung
- 2: Haubenkörper
- 2.1: Fettfilter
- 2.2: Leuchteinheiten
- 3: Kamin
- 3.1: Wandung
- 3.2: Halterung
- 4: Aufnahmeraum
- 5: Ausnehmung
- 6: Steg
- 7: Wasserbecken
- 8: Füllstandsanzeige
- 9: Beleuchtungseinrichtung
- 10: Entkeimungslampe
- 11: Lüftereinheit
- 12: Aktivkohlefilter
- 13: Pollenfilter

## Patentansprüche

1. Luftreinigungseinrichtung, insbesondere zur Belüftung einer Kochfläche, mit einem Haubenkörper (2) und einem am Haubenkörper (2) angeordneten Kamin (3) mit einer Wandung (3.1),
**dadurch gekennzeichnet,**
**dass** im Kamin (3) ein Aufnahmeraum (4) zur Aufnahme zumindest einer Pflanze vorgesehen ist und
**dass** in der Wandung (3.1) des Kamins (3) zumindest eine Ausnehmung (5) eingebracht ist, durch die der Aufnahmeraum (4) von außen zugänglich ist.

2. Luftreinigungseinrichtung nach Anspruch 1, **dadurch gekennzeichnet, dass** der Kamin (3) rohrförmig ausgebildet ist und umfangsseitig mehrere Ausnehmungen (5) aufweist.

3. Luftreinigungseinrichtung nach Anspruch 2, **dadurch gekennzeichnet, dass** die Ausnehmungen (5) jeweils durch Stege (6) voneinander getrennt sind.

4. Luftreinigungseinrichtung nach einem der vorhergehenden Ansprüche, **dadurch gekennzeichnet, dass** am Boden des Aufnahmeraumes (4) ein schalenartiges Element (7) zur Aufnahme von Wasser angeordnet ist.

5. Luftreinigungseinrichtung nach Anspruch 4, **dadurch gekennzeichnet, dass** das schalenartige Element (7) eine Füllstandsanzeige (8) aufweist.

6. Luftreinigungseinrichtung nach einem der vorhergehenden Ansprüche, **dadurch gekennzeichnet, dass** oberhalb des Aufnahmeraums (4) eine Beleuchtungseinrichtung (9), insbesondere zur Förderung des Pflanzenwachstums, angeordnet ist.

7. Luftreinigungseinrichtung nach Anspruch 6, **dadurch gekennzeichnet, dass** die Beleuchtungseinrichtung (9) aus einer Vielzahl von Leuchtdioden besteht.

8. Luftreinigungseinrichtung nach Anspruch 6 oder 7, **dadurch gekennzeichnet, dass** eine Zeitschaltuhr zur Steuerung der Beleuchtungseinrichtung (9) vorgesehen ist.

9. Luftreinigungsvorrichtung nach einem der vorhergehenden Ansprüche, **gekennzeichnet durch** die Ausbildung für den Umluftbetrieb.

10. Luftreinigungsvorrichtung nach einem der vorhergehenden Ansprüche, **dadurch gekennzeichnet, dass** eine Entkeimungseinrichtung, beispielsweise eine Entkeimungslampe (10) vorgesehen ist.
